# EUROPEAN PATENT SPECIFICATION

(11) **EP 3 543 343 B1**
(45) Date of publication and mention of the grant of the patent: **29.04.2020**
(21) Application number: 19162010.3
(22) Date of filing: 11.03.2019
(51) Int. Cl.: C12P 7/10, C12P 19/02, C12P 19/14

(54) **TWO-STAGE SIMULTANEOUS SACCHARIFICATION AND CO-FERMENTATION FOR PRODUCING ETHANOL FROM LIGNOCELLULOSE**
ZWEISTUFIGE SIMULTANE VERZUCKERUNG UND CO-FERMENTATION ZUR PRODUKTION VON ETHANOL AUS LIGNOCELLULOSE
SACCHARIFICATION ET CO-FERMENTATION SIMULTANÉES EN DEUX ÉTAPES POUR LA PRODUCTION D'ÉTHANOL À PARTIR DE LIGNOCELLULOSE

(30) Priority: 12.03.2018 IN 201821008982
(43) Date of publication of application: 25.09.2019
(73) Proprietor: INDIAN OIL CORPORATION Ltd., Mumbai 400 051 (IN); Department of Biotechnology, New Delhi 110 003 (IN)
(72) Inventor: SHARMA, Ajay Kumar, 121007 Haryana (IN); SWAIN, Manas Ranjan, 121007 Haryana (IN); SINGH, Ajit, 121007 Haryana (IN); MATHUR, Anshu Shankar, 121007 Haryana (IN); GUPTA, Ravi Prakash, 121007 Haryana (IN); TULI, Deepak, 121007 Haryana (IN); PURI, Suresh Kumar, 121007 Haryana (IN); RAMAKUMAR, Sankara Sri Venkata, 121007 Haryana (IN)
(74) Representative: De Gregori, Antonella

(56) References cited:
- JIN, M. ET AL.: "Two-step SSCF to convert AFEX-treated switchgrass to ethanol using commercial enzymes and Saccharomyces cerevisiae 424A(LNH-ST)", BIORESOURCE TECHNOLOGY, vol. 101, no. 21, 30 June 2010 (2010-06-30), pages 8171-8178, XP027134523,

## Description

### FIELD OF THE INVENTION

The present invention relates to a process for production of ethanol from lignocellulosic biomass.

### BACKGROUND OF THE INVENTION

Simultaneous Saccharification fermentation/co-fermentation (SSF/SSCF) removes sugar inhibition on enzymatic hydrolysis thus increases the hydrolysis sugar yield and reduces contamination risk. Moreover, SSF/SSCF reduces the overall reaction time and reactor volume (Kristensen et al., 2009). SSF/SSCF sacrifices the optimal conditions for both enzymatic hydrolysis and fermentation. Typically enzymatic hydrolysis and fermentation in SSF system the temperature is kept at 37-42°C as a compromise for better enzymatic hydrolysis and fermentation (Dien et al., 2003b). In addition, SSF/SSCF introduces a new inhibitor (ethanol) for enzymatic hydrolysis. But the inhibitory effect from ethanol is much lower compared to cellobiose or glucose (Taherzadeh & Karimi, 2007).

The major advantage of Separate Hydrolysis and fermentation /co-fermentation (SHF/SHCF) compared to SSF/SSCF is that enzymatic hydrolysis and fermentation can be carried out at their own optimal conditions (Taherzadeh & Karimi, 2007). However, enzymes during hydrolysis is easily inhibited by its end-products (sugars), especially during high solid loading enzymatic hydrolysis (Kristensen et al., 2009; Philippidis & Smith, 1995), which led to sluggish hydrolysis and resulted into enhanced hydrolysis time and high enzyme loading to achieve high sugar conversions. Another problem of this process is the high risk of contamination during enzymatic hydrolysis due to the long reaction time and high sugar concentrations (Taherzadeh & Karimi, 2007). Enzymatic hydrolysis is the limiting step for SHF, which determines the overall ethanol yield (Lau & Dale, 2009).

US 20060014260 disclose a simultaneous saccharification and fermentation (SSF) process for the bioconversion of cellulose into ethanol. It discloses that the reaction mixture comprises of slurry comprising cellulosic substrate, an enzyme and a fermentation agent. The reaction mixture is treated at a temperature between about 30° C and 48° C and a pH between about 4.0 and 6.0, along with agitation for a period of about 30 minutes to several hours or days.

US 20100268000 A1 discloses method for producing one or more fermentation end-products by fermenting a lignocellulosic biomass comprising hexose and pentose saccharides. Further it discloses using *Saccharomyces cerevisiae* along with fermentation medium supplement selected from the group consisting of a fatty acid, a surfactant, a chelating agent, vitamins, minerals, pH modifiers, yeast extract, and salts such as ammonium salts and salts of magnesium.

The document Jin, M. ET AL. "Two-step SSCF to convert AFEX-treated switchgrass to ethanol using commercial enzymes and Saccharomyces cerevisiae 424A(LNH-ST)" BIORESOURCE TECHNOLOGY 101 (2010) 8171-81-78 discloses a process for production of ethanol from a lignocellulosic biomass comprising:
(i) adding slurry of a pre-treated lignocellulosic biomass comprising C5 and C6 sugars in a fermenter system,
(ii) selectively fermenting mainly C5 sugars by incubating the pretreated lignocellulosic biomass with a small dose of cellulase enzyme, co-fermenting microorganism and nutrient to obtain ethanol,
(iii) adding a higher dose of cellulase enzyme,
(iv) allowing the fermenter system to cool to a temperature of 30°C, and
(v) selectively fermenting C6 sugars to obtain ethanol.

### SUMMARY OF THE INVENTION

In present invention of modified SSCF process, enzymatic hydrolysis is preceded by mainly C5 sugar fermentation and low enzymatic hydrolysis and succeeds by mainly C6 sugar fermentation at different temperature and duration. This resulted into higher ethanol titer at short time of combined hydrolysis and fermentation. Present invention is advantageous over conventional SSCF because initial free sugars and oligosaccharides in the pretreated biomass was targeted for fermentation which reduces the enzymatic feedback inhibition and optimum temperature were used for hydrolysis and fermentation, which resulted into higher ethanol yield at low dose of enzyme at short time interval.

In conventional fermentation practice glucose concentration is always higher than xylose concentration. So in this situation, yeast mostly prefers glucose fermentation which ultimately reduces the xylose fermentation efficiency and prolongs the xylose fermentation time.

In the present study free xylose and other oligosaccharides are targeted initial stage of fermentation which ultimately reduces enzymatic feedback inhibition. Due to which enzyme concentration was reduced to half than the conventional practice and further reduced fermentation time to one third and 30 h less than separate hydrolysis and fermentation (SHF) and conventional SSCF, respectively. This makes the process more acceptable for commercial practice.

Accordingly, present invention provides a process for production of ethanol from a lignocellulosic biomass comprising;
(i) adding slurry of a pre-treated lignocellulosic biomass comprising C5 and C6 sugars in a fermenter system;
(ii) selectively fermenting mainly C5 sugars by incubating the pretreated lignocellulosic biomass with a cellulase enzyme, co-fermenting microorganism and nutrient to obtain ethanol;
(iii) hydrolysing by heating the fermenter system to 48-55°C for a period of 18 to 24 hours;
(iv) allowing the fermenter system to cool to a temperature of 35-37°C; and
(v) selectively fermenting C6 sugars by inoculating the system with a second dose of co-fermenting microorganism to obtain ethanol.

Also disclosed is a process for production of ethanol from a lignocellulosic biomass comprising;
(i) adding whole slurry of an acid pre-treated lignocellulosic biomass comprising C5 and C6 sugars in a fermenter system;
(ii) selectively fermenting mainly C5 sugars by incubating the pretreated lignocellulosic biomass with a cellulase enzyme, co-fermenting microorganism and nutrient to obtain ethanol;
(iii) hydrolysing by heating the fermenter system to 48-55°C for a period of 18 to 24 hours;
(iv) allowing the fermenter system to cool to a temperature of 35-37°C; and
(v) selectively fermenting C6 sugars by inoculating the system with a second dose of co-fermenting microorganism to obtain ethanol.

In one of the feature of the present invention, the fermentation of C5 sugar is carried out for 16-20 hours at any temperature which favors fermentation over hydrolysis, when the xylose concentration is reduced to 6-7 g/l in fermentation broth the temperature of process is increased to 33 and 35°C gradually and incubated at 2h in each temperature for better hydrolysis and fermentation.

In a further feature of the present invention, the fermentation of C5 sugar is carried out at temperature in the range of 30°C-35°C.

In another feature of the present invention, the fermentation of C6 sugar is carried out for 6 to 10 hours at any temperature which favors fermentation over hydrolysis.

In a further feature of the present invention, the fermentation of C6 sugar is carried out at temperature in the range of 35°C-37°C.

In yet another feature of the present invention, the pre-treated biomass slurry is added in the fermenter system of step (i) without any detoxification.

In still another feature of the present invention, the process for production of ethanol from a lignocellulosic biomass additionally comprising adjusting pH of the slurry of step (i) to 5-5.5 with a pH adjuster.

In yet another feature of the present invention, the pH adjuster is selected from aqueous ammonium hydroxide, NaOH, KOH, and CaCO₃ or substance which is alkaline in nature and increases pH.

In still another feature of the present invention, the nutrient is ammonium sulphate, MgSO₄ or any other magnesium or ammonium salts.

In still another feature of the present invention, the cellulase enzyme is from fungal origin, β-glucosidase along with other accessory enzyme, wherein:
(i) the cellulase enzyme of fungal origin is composed of Cellobiohydrolase I and II; and
(ii) the other accessory enzymes is selected from xylanase, β-xyloxidase, arabinofuranosidase, and pectinse.

In yet another feature of the present invention, the co-fermenting (C6 and C5 sugar) microorganism is selected from *Saccharomyces cerevisiae, Pichia* sp., *Candida* sp., and *E. coli* or any ethanogenic co-fermenting microorganism.

In yet another feature of the present invention, the C5 sugar is selected from xylose and C6 sugar is selected from glucose.

In yet another feature of the present invention, the fermentation of C6 sugar is stopped after 6 to 10 hours of fermentation.

In still another feature of the present invention, optionally other nutrient is used to enhance the final ethanol concentration and the other nutrient is selected from yeast extract, peptone and ammonium sulphate or any other nitrogen source for microorganism.

In still another feature of the present invention, the lignocellulosic biomass is selected from straw, wheat straw, sugarcane bagasse, cotton stalk, barley stalk, bamboo or any agriculture residues which contain cellulose or hemicellulose or both.

In one of the feature, present invention provides a process for production of ethanol from a lignocellulosic biomass comprising:
(i) adding a pre-treated biomass slurry comprising C5 and C6 sugars without any detoxification in a fermenter system;
(ii) adjusting pH of the slurry of step (i) to 5-5.5 with aqueous ammonium solution to obtain a pH adjusted slurry;
(iii) fortifying the pH adjusted slurry with MgSO₄ in amount of 3-5 g/l, along with cellulase enzyme and co-fermenting microorganism;
(iv) adding water to the slurry of step (iii) to maintain 5 to 20 weight % Total solid (TS) in the slurry;
(v) incubating the slurry of step (iv) at 30°C-35°C for 16-20 hours for a selectively fermenting C5 sugars with 200-250 rpm when free xylose in the slurry to comes down to 6-7 g/l from 30 to 35 g/l;
(vi) hydrolysing by heating the fermenter system to 48-55°C at ramping of 3 to 4°C per 20-25 minutes, and then the process is allowed to maintain the temperature 48°C-55°C for 18-24 hours;
(vii) allowing the fermenter system to cool down to a temperature 35-37°C; and
(viii) selectively fermenting C6 sugars by inoculating the system with a second dose of co-fermenting microorganism to obtain ethanol.

Further disclosed is a process for production of ethanol from a lignocellulosic biomass comprising:
(i) adding a pre-treated biomass slurry comprising C5 and C6 sugars without any detoxification in a fermenter system;
(ii) adjusting pH of the slurry of step (i) to 5-5.5 with aqueous ammonium solution to obtain a pH adjusted slurry;
(iii) fortifying the pH adjusted slurry with MgSO₄ in amount of 3-5 g/l, along with cellulase enzyme and co-fermenting microorganism;
(iv) adding water to the slurry of step (iii) to maintain desired Total solid (TS) in the slurry;
(iv) incubating the slurry of step (iv) at 30°C-35°C for 16-20 hours for a selectively fermenting C5 sugars with 200-250 rpm when free xylose in the slurry to comes down to 6-7 g/l from 30 to 35 g/l; while temperatures increasing, fermentor is hold at 30 and 33°C for 2 hours to increase rate of hydrolysis and fermentation;
(v) hydrolysing by heating the fermenter system to 48-55°C at ramping of 3 to 4°C per 20-25 minutes, and then the process is allowed to maintain the temperature 48°C-55°C for 18-24 hours;
(iv) allowing the fermenter system to cool down to a temperature 35-37°C; and
(vi) selectively fermenting C6 sugars by inoculating the system with a second dose of co-fermenting microorganism to obtain ethanol.

### BRIEF DESCRIPTION OF DRAWINGS

Fig. 1 illustrates flowchart depicting a process for production of ethanol from a lignocellulosic biomass according to an embodiment of the present disclosure.
Fig. 2 illustrates results of invented modified SSCF using Co-fermenting *S. cerevisiae* and commercial enzyme (3.3 FPU/TS);
Fig. 3 illustrates results of conventional SSCF using *S. cerevisiae* and commercial enzyme (7 FPU/TS); and
Fig. 4 shows illustration of ethanol yield (%) of the modified SSCF process and conventional SSCF process for ethanol production

### DETAILED DESCRIPTION OF THE INVENTION

While the invention is susceptible to various modifications and alternative forms, specific embodiment thereof will be described in detail below.

### Definition:

For the purposes of this invention, the following terms will have the meaning as specified therein:
"Pre-treated biomass" or "Pretreatment of biomass" used herein clears away physical and chemical barriers that make native biomass recalcitrant and exposes cellulose for better enzymatic hydrolysis. In most of the pretreatment, chemical (acid or alkali) and physical (high temperature or pressure) parameters are used individually or in mixed manner to remove barriers for enzymatic hydrolysis and improve the enzymatic digestibility.

"Detoxification" used herein is the process where the inhibitors (toxic compound such hydroxymethyl furfural, furfural, acetic acids, formic acids etc.) produced during the pretreatment process are removed or neutralized from pre-treated biomass by chemical, physical or biological process.

"Cellulase enzyme" used herein is a mixed form of enzyme which is mostly composed of exo-hydrolase, endo-hydrolase and beta-glucosidase. This enzyme was mostly produced from fungal sources. Cellulase breaks down the cellulose molecule into monosaccharide and shorter polysaccharides or oligosaccharides. In the present invention the cellulase enzyme is selected from commercial available cellulase enzymes which are suitable for the purposes. More particularly commercial available cellulase enzyme CTec3 is used in the present invention.

"Free sugar" used herein is the monomeric form of sugar which are produced from the lignocellulosic biomass during the pretreatment. Free sugar in this process composed of mainly glucose and xylose.

"C5 sugars" used herein C5 sugars represented for Xylose. "Free C5 sugar" used herein is sugar (mostly xylose) released from the hemicelluloses during the pretreatment and some part in enzymatic hydrolysis.

"C5 fermentation" used herein is Xylose fermentation into ethanol.

"C6 sugar" used herein represents glucose.

"C6 fermentation" used herein is Glucose fermentation into ethanol.

"Nutrient" used herein is Ammonium hydroxide and MgSO₄. Ammonium hydroxide used in this process has dual activity, it adjust the pH of the sulphuric acid (H₂SO₄) pretreated biomass and simultaneously converted to ammonium sulphate (ammonium ion (NH₄⁺) combined with free sulphates (SO4⁻²) ions released from the sulphuric acid during the pretreatment. Ammonium sulphate ((NH4)₂SO₄) acts as a nitrogen source for yeast during fermentation. Another salt MgSO₄ used in fermentation where, Mg⁺² act as an essential enzyme cofactor and act as key structural component of most biological pathways. During fermentation Mg⁺² plays a major role for proper functioning of fermenting enzymes in yeast.

The present invention discloses a method for production of ethanol from lignocellulosic biomass. In the present invention, free C5 sugar in pre-treated biomass is targeted first along with available low concentration of glucose for fermentation followed by enzymatic hydrolysis and C6 fermentation in sequential manner.

Xylan and Glucan are polymer of xylose and glucose respectively collectively called as holocellulose in lignocellulosic biomass. As per the physical property xylan and glucan are amorphous and crystalline in nature respectively. Due to the physical property, xylan gets breaks down to xylose when lignocellulosic biomass subjected to acid pretreatment but most of the glucan remain un-reacted. So in this process when the pretreated biomass is taken for fermentation, free form of xylose (30-35g/L) (breakdown xylan) are present in the biomass which is targeted firstly by the co-fermenting microorganism for fermentation in presence of very less amount of glucose (< 8-10 g/L, which is released during the pretreatment) at 30-35°C. The fermentation temperature is not adequate enough for the enzyme to breakdown of the glucan to glucose efficiently. So due to this the co-fermenting microorganism mostly targeted xylose (C5) sugar at the initial stage of fermentation.

The process, in accordance with the present invention, brings the C5 concentration about to dryness and brings down the total process time (both hydrolysis and fermentation) to 46h which is about 1/3 of the conventional SHF (total process time 120h which include 72 h Hydrolysis and 48 h Fermentation). Overall ethanol productivity is much higher than conventional SSCF process.

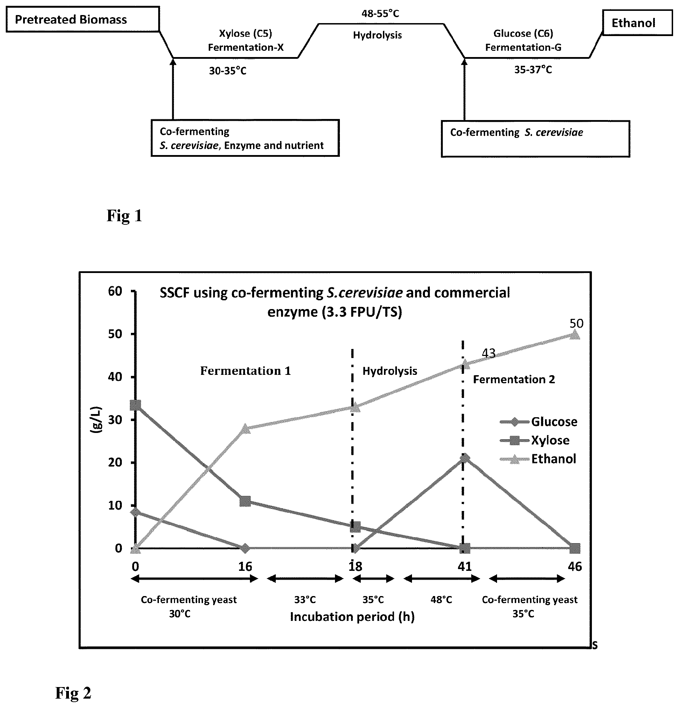

In accordance with the present invention, a method for production of ethanol from lignocellulosic biomass (see **Fig 1**) is disclosed, comprising:
1. Addition of acid pre-treated biomass slurry without any detoxification in the fermenter;
2. The pH of the slurry was adjusted to 5-5.5 with aqueous ammonium solution. The pH adjusted slurry was fortified with 3-5 g/l MgSO₄, cellulase enzyme and co-fermenting *Saccharomyces cerevisiae;* the co-fermenting yeast is essential to use in this process to utilize both pentose and hexose sugar in the fermentation broth.
3. Appropriate water is added in to fermenter to adjust the final Total solid (TS) loading at 20% of biomass. The whole process is then incubated at 30°C-35°C for 16-20 hours for the fermentation with 200 rpm;
4. When the concentration of free xylose (free xylose is produced during the process of acid pretreatment and readily available for fermentation during fermentation process) in the slurry comes down to 3-5 g/l, the temperature of the process was slowly increased to 48-55°C at ramping of 3 to 4°C per 20-25 min., then the process is allowed to maintain about 48-55°C temperature for 18-24 hours. After this incubation the system was allowed to cool down to temperature 35-37°C;
5. A second dose of co-fermenting *Saccharomyces cerevisiae* is inoculated to the system for the second stage of fermentation. The second fermentation was stopped after 6 to 10 hours of fermentation.

Having described the basic aspects of the present invention, the following examples illustrate specific embodiment thereof.

### Example 1

Pretreated biomass (slurry, TS approximately 24%) without any detoxification is introduced directly to the fermenter. The pH of the slurry was adjusted to 5.5 with aqueous ammonium solution (25% initial concentration). The pH adjusted slurry was fortified with 3 g/l MgSO₄, cellulase enzyme (Commercial enzyme, 3.3FPU/TS) and co-fermenting *Saccharomyces cerevisiae* (1g dry cell biomass/litre, xylose and glucose utilizing yeast). Required amount of water was added to the process to adjust the final biomass concentration to 20%. The whole process was incubated at 30°C for 16 h for the fermentation with 200 rpm. When the free xylose concentration in the slurry comes near to 6-7g/l, the temperature of the process was increased to 33°C and 35°C, incubated for 2h in each temperature for better hydrolysis and fermentation. After that temperature increased to 48°C. This step mainly required for rapid releases of glucose sugar from cellulose which converted simultaneously with hydrolysis to ethanol by yeast biomass. As the temperature was reached at desired target the process was allowed to maintain the required temperature (48°C) for 23h for better enzymatic hydrolysis. After this incubation the system was allowed to cool down to temperature 35°C. A second dose of co-fermenting *S. cerevisiae* (1g dry cell biomass/liter) was inoculated to the system for the second stage of fermentation. The second fermentation was stopped after 6 h of fermentation. This process took 46h incubation including fermentation and enzymatic hydrolysis. The results of this experiment are represented by **Fig 2****.**

| Solid Loading in Fermentation | 20% |
|---|---|
| Mode of Fermentation | SSCF, Single yeast strain co-fermenting *Saccharomyces cerevisiae* (2g/L( used in both fermentation |
| Enzyme loading (FPU/g) and Sources | 3.3, Commercial enzyme |
| Residual Xylose (g/L) | 1.30 |
| Ethanol Concentration (g/L) at 46 h | 50 |
| Ethanol Yield (%) | 71 |
| Specific Productivity (Q) g/L/h | 1.08 |

### Example 2

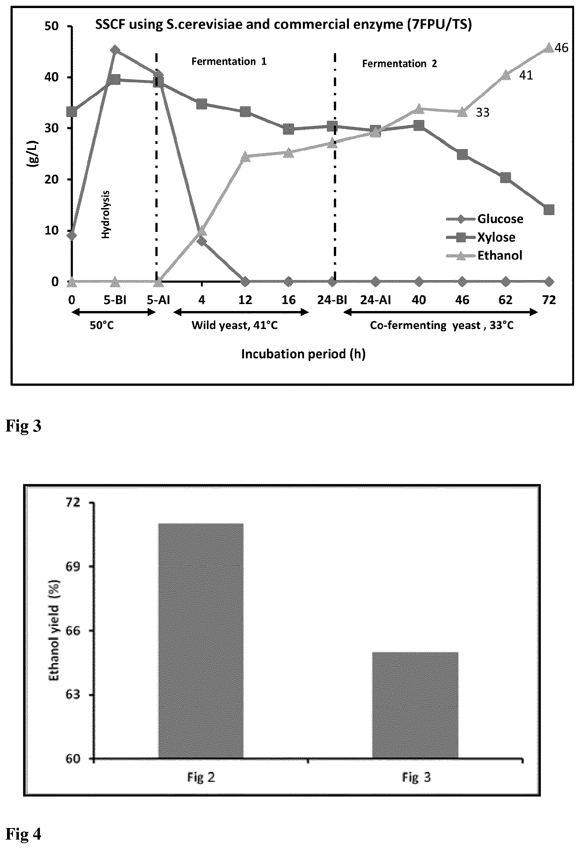

Using conventional SSCF approach of ethanol production from pretreated biomass, saccharification at 50°C for 5h and followed by fermentation and hydrolysis at 41°C by a moderately thermo tolerant wild yeast *S. cerevisiae* up to 24h. After this fermentation another yeast co-fermenting *S. cerevisiae* was inoculated to the fermentation process. In this approach the xylose utilization after the glucose fermentation was comparatively slow as compare to the above process and about 10 g/l residual xylose was observed after 72 h. This process of fermentation brings the lower ethanol titer after the 72 h of fermentation using even higher enzyme dosage. The results of this experiment are represented by **Fig 3****.**

| Solid Loading in Fermentation | 20% |
|---|---|
| Mode of Fermentation | SSCF, 1^{st} wild type *Saccharomyces cerevisiae* (1 g/l), 2^{nd} co-fermenting *Saccharomyces cerevisiae* (1g/l) |
| Enzyme loading (FPU/g) and Sources | 7, Commercial enzyme |
| Residual Xylose (g/L) | 9.98 |
| Ethanol Concentration (g/L) at 72 h | 46 |
| Ethanol Yield (%) | 65 |
| Specific Productivity (Q) g/L/h | 0.64 |

Figure 4 represents the comparative ethanol yield (%) from Example 1 (Modified SSCF) and 2(Conventional SSCF process).

### References

1. Krishnan, C., Sousa, L.D., Jin, M.J., Chang, L.P., Dale, B.E., Balan, V. 2010. Alkali-based AFEX Pretreatment for the conversion of sugarcane bagasse and cane leaf residues to ethanol. Biotechnology and Bioengineering, 107(3), 441-450.
2. Dien, B.S., Cotta, M.A., Jeffries, T.W. 2003. Bacteria engineered for fuel ethanol production: current status. Applied Microbiology and Biotechnology, 63(3), 258-266.
3. Taherzadeh, M.J., Karimi, K. 2007. Enzyme-based hydrolysis processes for ethanol from lignocellulosic materials: a review. Bioresources, 2(4), 707-738.
4. Lau, M.W., Dale, B.E. 2009. Cellulosic ethanol production from AFEX-treated corn stover using Saccharomyces cerevisiae 424A (LNH-ST). Proceedings of the National Academy of Sciences of the United States of America, 106(5), 1368-1373.

## Claims

1. A process for production of ethanol from a lignocellulosic biomass comprising;
(i) adding slurry of a pre-treated lignocellulosic biomass comprising C5 and C6 sugars in a fermenter system;
(ii) selectively fermenting mainly C5 sugars by incubating the pretreated lignocellulosic biomass with a cellulase enzyme, co-fermenting microorganism and nutrient to obtain ethanol;
(iii) hydrolysing by heating the fermenter system to 48-55°C for a period of 18 to 24 hours;
(iv) allowing the fermenter system to cool to a temperature of 35-37°C; and
(v) selectively fermenting C6 sugars by inoculating the system with a second dose of co-fermenting microorganism to obtain ethanol.

2. The process as claimed in claim 1, wherein the fermentation of C5 sugar is carried out for 16-20 hours at any temperature which favors fermentation over hydrolysis, when the xylose concentration is reduced to 6-7 g/l in fermentation broth the temperature of process is increased to 33 and 35°C gradually and incubated at 2h in each temperature for better hydrolysis and fermentation.

3. The process as claimed in claim 2, wherein the fermentation of C5 sugar is carried out at temperature in the range of 30°C-35°C.

4. The process as claimed in claim 1, wherein the fermentation of C6 sugar is carried out for 6 to 10 hours at any temperature which favors fermentation over hydrolysis.

5. The process as claimed in claim 4, wherein the fermentation of C6 sugar is carried out at temperature in the range of 35°C-37°C.

6. The process as claimed in claim 1, wherein the pre-treated biomass slurry is added in the fermenter system of step (i) without any detoxification.

7. The process as claimed in claim 1, additionally comprising adjusting pH of the slurry of step (i) to 5-5.5 with a pH adjuster.

8. The process as claimed in claim 7, wherein the pH adjuster is selected from aqueous ammonium hydroxide, NaOH, KOH, and CaCO₃ or substance which is alkaline in nature and increases pH.

9. The process as claimed in claim 1, wherein the nutrient is ammonium sulphate, MgSO₄ or any other magnesium or ammonium salts.

10. The process as claimed in claim 1, wherein the cellulase enzyme is from fungal origin, β-glucosidase along with other accessory enzyme, wherein:
(i) the cellulase enzyme of fungal origin is composed of cellobiohydrolase I and II; and
(ii) the other accessory enzymes is selected from xylanase, β-xyloxidase, arabinofuranosidase, and pectinse.

11. The process as claimed in claim 1, wherein the co-fermenting microorganism is selected from *Saccharomyces cerevisiae, Pichia* sp., *Candida* sp., and *E. coli* or any ethanogenic co-fermenting microorganism.

12. The process as claimed in claim 1, wherein the C5 sugar is selected from xylose and C6 sugar is selected from glucose.

13. The process as claimed in claim 1, wherein the lignocellulosic biomass is selected from straw, wheat straw, sugarcane bagasse, cotton stalk, barley stalk, bamboo or any agriculture residues which contain cellulose or hemicellulose or both.

14. 13. A process for production of ethanol from a lignocellulosic biomass comprising:
(i) adding a pre-treated biomass slurry comprising C5 and C6 sugars without any detoxification in a fermenter system;
(ii) adjusting pH of the slurry of step (i) to 5-5.5 with aqueous ammonium solution to obtain a pH adjusted slurry;
(iii) fortifying the pH adjusted slurry with MgSO₄ in amount of 3-5 g/l, along with cellulase enzyme and co-fermenting microorganism;
(iv) adding water to the slurry of step (iii) to maintain 5 to 20 weight % Total solid (TS) in the slurry;
(v) incubating the slurry of step (iv) at 30°C-35°C for 16-20 hours for a selectively fermenting C5 sugars with 200-250 rpm when free xylose in the slurry to comes down to 6-7 g/l from 30 to 35 g/l;
(vi) hydrolysing by heating the fermenter system to 48-55°C at ramping of 3 to 4°C per 20-25 minutes, and then the process is allowed to maintain the temperature 48°C-55°C for 18-24 hours;
(vii) allowing the fermenter system to cool down to a temperature 35-37°C; and
(viii) selectively fermenting C6 sugars by inoculating the system with a second dose of co-fermenting microorganism to obtain ethanol.

## Patentansprüche

1. Verfahren zur Ethanolproduktion aus Lignocellulose-Biomasse, umfassend:
(i) Zugabe einer Aufschlämmung aus vorbehandelter Lignocellulose-Biomasse, umfassend C5- und C6-Zuckern in ein Fermentationssystem
(ii) selektive Fermentation hauptsächlich von C5-Zuckern durch Inkubation der vorbehandelten Lignocellulose-Biomasse mit einem Cellulase-Enzym, cofermentierenden Mikroorganismen und Nährstoff, um Ethanol zu erhalten;
(iii) Hydrolyse durch Erhitzen des Fermentationssystems auf 48-55°C über einen Zeitraum von 18 bis 24 Stunden;
(iv) das Fermentationssystem auf eine Temperatur von 35-37°C abkühlen lassen; und
(v) selektive Fermentation von C6-Zuckern durch Inokulation des Systems mit einer zweiten Dosis von cofermentierenden Mikroorganismen, um Ethanol zu erhalten.

2. Verfahren wie in Anspruch 1 beansprucht, wobei die Fermentation von C5-Zucker für 16-20 Stunden bei jeder beliebigen Temperatur durchgeführt wird, welche die Fermentation über Hydrolyse begünstigt. Wenn die Xylose-Konzentration auf 6 -7 g/l in der Fermentationsbrühe gesunken ist, steigt die Temperatur des Prozesses allmählich auf 33 und 35°C und wird, zur besseren Hydrolyse und Fermentation, für 2h bei jeder Temperatur inkubiert.

3. Verfahren wie in Anspruch 2 beansprucht, wobei die Fermentation von C5-Zucker bei einer Temperatur im Bereich von 30°C-35°C durchgeführt wird.

4. Verfahren wie in Anspruch 1 beansprucht, wobei die Fermentation von C6-Zucker während 6 bis 10 Stunden bei jeder Temperatur durchgeführt wird, welche die Fermentation über Hydrolyse begünstigt.

5. Verfahren wie in Anspruch 4 beansprucht, wobei die Fermentation von C6-Zucker bei einer Temperatur im Bereich von 35°C-37°C durchgeführt wird.

6. Verfahren wie in Anspruch 1 beansprucht, wobei die vorbehandelte Biomasseaufschlämmung dem Fermentationssystem von Schritt (i) ohne jegliche Detoxifizierung zugegeben wird.

7. Verfahren wie in Anspruch 1 beansprucht, zusätzlich umfassend die Einstellung des pH-Wertes der Aufschlämmung von Schritt (i) auf 5-5.5 mit einem pH-Einsteller.

8. Verfahren wie in Anspruch 7 beansprucht,
wobei der pH-Einsteller ausgewählt ist aus wässrigem Ammonium Hydroxid, NaOH, KOH, und CaCO₃ oder einer Substanz, die in Natur alkalisch ist und den pH-Wert erhöht.

9. Verfahren wie in Anspruch 1 beansprucht, wobei der Nährstoff Ammoniumsulfat, MgSO₄ oder irgendwelche andere Magnesium- oder Ammoniumsalze ist.

10. Verfahren wie in Anspruch 1 beansprucht, wobei das Cellulase-Enzym ursprünglich aus einem Pilz stammt, β-Glucosidase zusammen mit anderen akzessorischen Enzymen, wobei:
(i) das aus einem Pilz stammende Cellulase-Enzym zusammengesetzt ist aus Cellobiohydrolase I und II; und
(ii) das andere akzessorische Enzym ausgewählt ist aus Xylanase, β-Xyloxidase, Arabinofuranosidase und Pektinase.

11. Verfahren wie in Anspruch 1 beansprucht, wobei der cofermentierende Mikroorganismus ausgewählt ist aus *Saccharomyces cerevisiae, Pichia* sp., *Candida* sp., und *E. coli* oder jegliche ethanogene cofermentierende Mikroorganismen.

12. Verfahren wie in Anspruch 1 beansprucht, wobei der C5-Zucker ausgewählt ist aus Xylose und der C6-Zucker ausgewählt ist aus Glucose.

13. Verfahren wie in Anspruch 1 beansprucht, wobei die Lignocellulose-Biomasse ausgewählt ist aus Stroh, Weizenstroh, Zuckerrohr-Bagasse, Baumwollstängel, Gerstenstängel, Bambus oder jeglichen landwirtschaftlichen Reststoffen, die Zellulose oder Hemizellulose bzw. beide enthalten.

14. Verfahren zur Ethanolproduktion aus Lignocellulose-Biomasse, umfassend:
(i) Zugabe einer vorbehandelten Biomasseaufschlämmung umfassend C5- und C6-Zuckern ohne jegliche Detoxifizierung in einem Fermentationssystem;
(ii) Einstellung des pH-Wertes der Aufschlämmung von Schritt (i) auf 5-5.5 mit wässriger Ammonium-Lösung, um eine pH-eingestellte Aufschlämmung zu erhalten;
(iii) Verstärken der pH-eingestellten Aufschlämmung mit MgSO₄ in einer Menge von 3-5 g/1 zusammen mit Cellulase-Enzym und cofermentierenden Mikroorganismen;
(iv) Zugabe von Wasser in die Aufschlämmung von Schritt (iii) um 5 bis 20 Gew.-% an Gesamtfeststoff (TS) in der Aufschlämmung zu erhalten;
(v) Inkubieren der Aufschlämmung von Schritt (iv) auf 30°C-35°C über einen Zeitraum von 16-20 Stunden zur selektiven Fermentation von CS-Zuckern bei 200-250 Umin, wenn freie Xylose in der Aufschlämmung von 30 bis 35 g/l auf 6-7 g/l absinkt;
(vi) Hydrolysieren durch Erhitzen des Fermentationssystems auf 48-55°C bei einer Steigung von 3 bis 4°C 20 -25 Minuten lang; und woraufhin dem Verfahren erlaubt ist, die Temperatur für 18-24 Stunden zwischen 48°C und 55°C zu halten;
(vii) das Fermentationssystem auf eine Temperatur von 35-37°C abkühlen lassen; und
(viii) selektive Fermentation von C6-Zuckern durch Inokulation des Systems mit einer zweiten Dosis von cofermentierenden Mikroorganismen, um Ethanol zu erhalten.

## Revendications

1. Un procédé de production d'éthanol à partir d'une biomasse lignocellulosique comprenant :
(i) le fait d'ajouter une bouillie d'une biomasse lignocellulosique prétraitée comprenant des sucres en C5 et C6 dans un système de fermentation ;
(ii) le fait de faire fermenter de façon sélective des sucres principalement en C5 par incubation de la biomasse lignocellulosique prétraitée avec une enzyme cellulase, un micro-organisme de co-fermentation et un nutriment pour obtenir de l'éthanol ;
(iii) le fait d'hydrolyser par chauffage du système de fermentation à 48-55 °C pendant une durée de 18 à 24 heures ;
(iv) le fait d'autoriser le système de fermentation à refroidir à une température de 35-37 °C ; et
(v) le fait de faire fermenter de façon sélective des sucres en C6 par inoculation du système avec une deuxième dose de micro-organisme de co-fermentation pour obtenir de l'éthanol.

2. Le procédé tel que revendiqué dans la revendication 1, dans lequel la fermentation de sucre en C5 est réalisée pendant 16-20 heures à n'importe quelle température qui favorise la fermentation par rapport à l'hydrolyse, lorsque la concentration en xylose est réduite à 6-7 g/l dans un bouillon de fermentation la température du procédé est augmentée à 33 et 35 °C progressivement et incubée à 2 h dans chaque température pour une meilleure hydrolyse et fermentation.

3. Le procédé tel que revendiqué dans la revendication 2, dans lequel la fermentation de sucre en C5 est réalisée à une température dans la gamme de 30 °C-35 °C.

4. Le procédé tel que revendiqué dans la revendication 1, dans lequel la fermentation de sucre en C6 est réalisée pendant 6 à 10 heures à n'importe quelle température qui favorise la fermentation par rapport à l'hydrolyse.

5. Le procédé tel que revendiqué dans la revendication 4, dans lequel la fermentation de sucre en C6 est réalisée à une température dans la gamme de 35 °C-37 °C.

6. Le procédé tel que revendiqué dans la revendication 1, dans lequel la bouillie de biomasse prétraitée est ajoutée dans le système de fermentation de l'étape (i) sans aucune détoxification.

7. Le procédé tel que revendiqué dans la revendication 1, comprenant en outre le fait d'ajuster le pH de la bouillie de l'étape (i) à 5-5,5 avec un ajusteur de pH.

8. Le procédé tel que revendiqué dans la revendication 7, dans lequel l'ajusteur de pH est sélectionné parmi l'hydroxyde d'ammonium aqueux, NaOH, KOH, et CaCO₃ ou une substance qui est de nature alcaline et augmente le pH.

9. Le procédé tel que revendiqué dans la revendication 1, dans lequel le nutriment est du sulfate d'ammonium, du MgSO₄ ou tous autres sels de magnésium ou d'ammonium.

10. Le procédé tel que revendiqué dans la revendication 1, dans lequel l'enzyme cellulase est d'origine fongique, β-glucosidase conjointement avec d'autres enzymes accessoires, dans lequel :
(i) l'enzyme cellulase d'origine fongique est composée de cellobiohydrolase I et Il ; et
(ii) les autres enzymes accessoires sont sélectionnées parmi la xylanase, la β-xyloxydase, l'arabinofuranosidase, et la pectine.

11. Le procédé tel que revendiqué dans la revendication 1, dans lequel le micro-organisme de co-fermentation est sélectionné parmi *Saccharomyces cerevisiae, Pichia sp., Candida sp.,* et *E. coli ou* tout micro-organisme de co-fermentation éthanogène.

12. Le procédé tel que revendiqué dans la revendication 1, dans lequel le sucre en C5 est sélectionné à partir de xylose et le sucre en C6 est sélectionné à partir de glucose.

13. Le procédé tel que revendiqué dans la revendication 1, dans lequel la biomasse lignocellulosique est sélectionnée parmi la paille, la paille de blé, la bagasse de canne à sucre, la tige de coton, la tige d'orge, le bambou ou tous résidus agricoles qui contiennent de la cellulose ou de l'hémicellulose ou les deux.

14. Un procédé de production d'éthanol à partir d'une biomasse lignocellulosique comprenant :
(i) le fait d'ajouter une bouillie de biomasse prétraitée comprenant des sucres en C5 et C6 sans aucune détoxification dans un système de fermentation ;
(ii) le fait d'ajuster le pH de la bouillie de l'étape (i) à 5-5,5 avec une solution aqueuse d'ammonium pour obtenir une bouillie à pH ajusté ;
(iii) le fait de fortifier la bouillie à pH ajusté avec du MgSO₄ à une concentration de 3-5 g/l, conjointement avec une enzyme cellulase et un micro-organisme de co-fermentation ;
(iv) le fait d'ajouter de l'eau à la bouillie de l'étape (iii) pour maintenir de 5 à 20 % en poids de matières solides totales (TS) dans la bouillie ;
(v) le fait d'incuber la bouillie de l'étape (iv) à 30 °C-35 °C pendant 16-20 heures pour une fermentation de façon sélective de sucres de type C5 avec 200-250 tours par minute lorsque le xylose libre dans la bouillie descend de 30-35 g/l à 6-7 g/l ;
(vi) le fait hydrolyser par chauffage du système de fermentation à 48-55 °C à une rampe de 3 à 4 °C par 20-25 minutes, et ensuite le procédé est autorisé à maintenir la température de 48 °C-55 °C pendant 18-24 heures ;
(vii) le fait d'autoriser le système de fermentation à refroidir à une température de 35-37 °C ; et
(viii) le fait de faire fermenter de façon sélective des sucres en C6 par inoculation du système avec une deuxième dose de micro-organisme de co-fermentation pour obtenir de l'éthanol.
